(19) Europäisches Patentamt / European Patent Office / Office européen des brevets

(11) **EP 3 895 599 A1**

(12) **EUROPEAN PATENT APPLICATION**
published in accordance with Art. 153(4) EPC

(43) Date of publication:
**20.10.2021 Bulletin 2021/42**

(51) Int Cl.:
**A61B 3/12** *(2006.01)*    **A61B 3/13** *(2006.01)*

(21) Application number: **19895248.3**

(86) International application number:
**PCT/JP2019/039521**

(22) Date of filing: **07.10.2019**

(87) International publication number:
**WO 2020/121633 (18.06.2020 Gazette 2020/25)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **10.12.2018 JP 2018230771**

(71) Applicant: NIKON CORPORATION
Minato-ku
Tokyo 108-6290 (JP)

(72) Inventors:
• **KAWASAKI, Tomohiro**
  **Tokyo 108-6290 (JP)**
• **OTSUBO, Yosuke**
  **Tokyo 108-6290 (JP)**
• **OTANI, Naoya**
  **Tokyo 108-6290 (JP)**
• **KONISHI, Kohki**
  **Tokyo 108-6290 (JP)**

(74) Representative: **Hoffmann Eitle**
**Patent- und Rechtsanwälte PartmbB**
**Arabellastraße 30**
**81925 München (DE)**

(54) **OPTICAL DEVICE, PROGRAM, CONTROL DEVICE, AND IMAGING SYSTEM**

(57)    An optical device includes: an imaging optical system configured to form an image of a subject; an imaging element configured to output an image signal of the image of the subject that is formed by the imaging optical system; a spatial modulation element configured to change a phase of a wavefront in a pupil of the imaging optical system; and a control unit configured to control the spatial modulation element on the basis of the image signal output by the imaging element.

FIG. 1

## Description

[Technical Field]

The present invention relates to an optical device, a program, a control device, and an imaging system.
Priority is claimed on Japanese Patent Application No. 2018-230771, filed December 10, 2018, the content of which is incorporated herein by reference.

[Background Art]

For example, an optical device such as a microscope disclosed in Patent Document 1 includes an illumination optical system and an imaging optical system. In such an optical device, there is a demand to improve imaging performance.

[Citation List]

[Patent Literature]

[Patent Document 1]
Japanese Unexamined Patent Application, First Publication No. 2015-72303

[Summary of Invention]

According to one aspect of the present invention, there is provided an optical device including: an imaging optical system configured to form an image of a subject; an imaging element configured to output an image signal of the image of the subject that is formed by the imaging optical system; a spatial modulation element configured to change a phase of a wavefront in a pupil of the imaging optical system; and a control unit configured to control the spatial modulation element on the basis of the image signal output by the imaging element.
According to one aspect of the present invention, there is provided a program for causing a computer of an optical device, which includes an imaging optical system configured to form an image of a subject, an imaging element configured to output an image signal of the image of the subject that is formed by the imaging optical system, and a spatial modulation element configured to change a phase of a wavefront in a pupil of the imaging optical system, to execute: an acquisition step of acquiring the image signal output by the imaging element; and a control step of controlling the spatial modulation element on the basis of the image signal acquired in the acquisition step.
According to one aspect of the present invention, there is provided a control device including a control unit configured to control a spatial modulation element changing a phase of a wavefront in a pupil of an imaging optical system, which forms an image of a subject, on the basis of an image signal that is a signal acquired by imaging the image of the subject formed by the imaging optical system using an imaging element.
According to one aspect of the present invention, there is provided an imaging system including: the control device described above; the imaging optical system described above; the above-described imaging element configured to output the image signal of the image of the subject formed by the imaging optical system described above; and the spatial modulation element described above.
According to one aspect of the present invention, there is provided a program causing a computer to execute: an acquisition step of acquiring an image signal that is a signal acquired by imaging an image of a subject formed by an imaging optical system forming the image of the subject using an imaging element; and a control step of controlling a spatial modulation element changing a phase of a wavefront in a pupil of the imaging optical system on the basis of the image signal acquired in the acquisition step.

[Brief Description of Drawings]

Fig. 1 is a diagram illustrating an example of an imaging system according to a first embodiment of the present invention.
Fig. 2 is a diagram illustrating an example of an image according to the first embodiment of the present invention.
Fig. 3 is a diagram illustrating an example of a wavefront state of a wavefront control device according to the first embodiment of the present invention.
Fig. 4 is a diagram illustrating an example of the configuration of a learning device according to the first embodiment of the present invention.

Fig. 5 is a diagram illustrating an example of the configuration of a control device according to the first embodiment of the present invention.

Fig. 6 is a diagram illustrating an example of a learning process according to the first embodiment of the present invention.

Fig. 7 is a diagram illustrating an example of optical system conditions according to the first embodiment of the present invention.

Fig. 8 is a diagram illustrating an example of a wavefront state according to the first embodiment of the present invention.

Fig. 9 is a diagram illustrating an example of imaging performance and weighting factors according to the first embodiment of the present invention.

Fig. 10 is a diagram illustrating an example of a control process for a wavefront control device using the control device according to the first embodiment of the present invention.

Fig. 11 is a diagram illustrating an example of the configuration of a learning device according to a modified example of the first embodiment of the present invention.

Fig. 12 is a diagram illustrating an example of a learning process according to a modified example of the first embodiment of the present invention.

Fig. 13 is a diagram illustrating the configuration of a control device according to a second embodiment of the present invention.

Fig. 14 is a diagram illustrating an example of a control process for a wavefront control device using the control device according to the second embodiment of the present invention.

Fig. 15 is a diagram illustrating an example of an adaptive learning process according to the second embodiment of the present invention.

[Description of Embodiments]

(First embodiment)

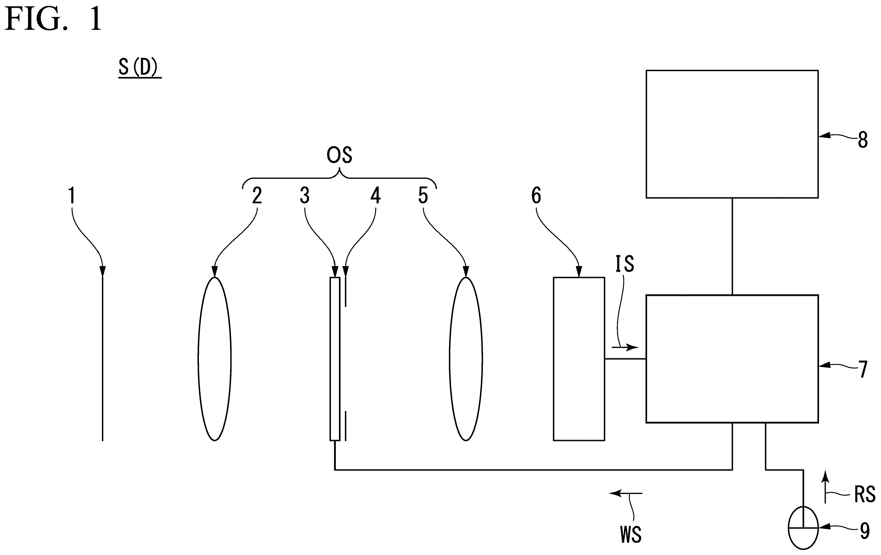

Hereinafter, embodiments of the present invention will be described in detail with reference to the drawings. Fig. 1 is a diagram illustrating an example of an imaging system S according to this embodiment. In addition, the imaging system S is an optical device D as well. The imaging system S (the optical device D) according to this embodiment is, for example, a microscope for ophthalmology. The imaging system S (the optical device D) may be a biological endoscope, an industrial endoscope, a medical endoscope, a monitoring camera, an in-vehicle camera, or the like.

The imaging system S includes an imaging optical system OS, a wavefront control device 3, an imaging element 6, a computer 7, a display unit 8, and an input unit 9.

The imaging optical system OS forms an image of a subject 1. The imaging optical system OS includes an objective lens system 2, a diaphragm 4, and an imaging lens system 5. The wavefront control device 3 is an example of a spatial modulation element.

A subject 1 is installed at a focal position of the objective lens system 2. Rays of light that have come out from the subject 1 are projected to the wavefront control device 3 in an afocal state. The wavefront control device 3 changes a phase of a wavefront in a pupil of the imaging optical system OS. The wavefront control device 3 is installed at a position conjugate with the diaphragm 4 or in the vicinity of the diaphragm 4 and thus has a pupil surface coinciding with that of the installation position of the wavefront control device 3. In other words, the wavefront control device 3 is disposed at a position conjugate with the pupil of the imaging optical system OS.

The wavefront control device 3 is, for example, a liquid crystal phase modulation element (liquid crystal on silicon (LCOS)). The wavefront control device 3 may be a micro electro mechanical systems (MEMS) mirror or a deformable mirror.

After being transmitted through or reflected on the wavefront control device 3, rays of light are collected at the imaging element 6 through the imaging lens system 5. The imaging element 6 is installed at the focal position of the imaging lens system 5. The imaging element 6 outputs an image signal IS of the image of a subject 1 formed by the imaging optical system OS. The image signal IS output by the imaging element 6 is transmitted to the computer 7 and is displayed on the display unit 8 as an image P.

The input unit 9 accepts an input from a user of the imaging system S. The user of the imaging system S designates a specific area RC at which the user desires to gaze in an area R included in the image P displayed on the display unit 8 through the input unit 9.

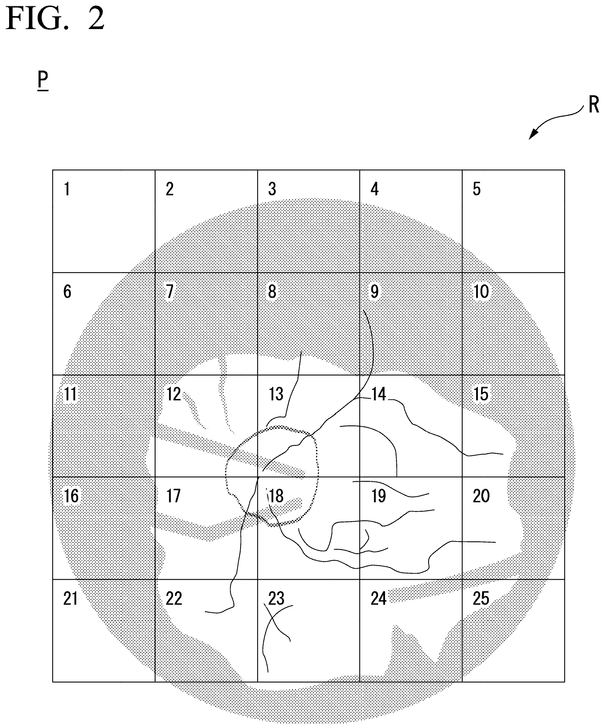

Here, the image P and the area R will be described with reference to Fig. 2.

Fig. 2 is a diagram illustrating an example of the image P according to this embodiment. The image P, for example, is an image of a retina during an operation. In this embodiment, the image P is divided into areas R1 to R25.

For example, the areas R1 to R25 are partial areas of the image P and are areas of squares having equal area respectively. The area R described above collectively refers to the areas R1 to R25.

The description of the configuration of the imaging system S will be continued with reference back to Fig. 1.

The input unit 9, for example, is a mouse. The input unit 9 may be a keyboard or a touch panel. The input unit 9 may be a stereoscopic display or a head mount display that detects a visual line of a user of the imaging system S. The input unit 9 may be a foot switch. The input unit 9 is a recognition device that recognizes a voice and gestures of the user of the imaging system S.

The computer 7 includes a learning device 7A, a control device 7B, an optical simulation device 7C, and a retina image database 7D. Each of the learning device 7A, the control device 7B, and the optical simulation device 7C is, for example, a module that is realized by a CPU of the computer 7 reading a program from a ROM and executing a process. The retina image database 7D is a storage device that is included in the computer 7.

The control device 7B controls the wavefront control device 3 on the basis of an image signal IS output by the imaging element 6, thereby improving imaging performance PY of a designated specific area RC. The learning device 7A learns the relationship between an image signal IS and a phase of a wavefront in a pupil of the imaging optical system OS in advance.

In this embodiment, the control device 7B controls the wavefront control device 3 on the basis of a wavefront state table T that is a result of learning performed by the learning device 7A.

Here, the wavefront state table T is a table in which a wavefront state $\theta$ and an imaging performance evaluation value Y are associated with each other on the basis of machine learning for each piece of optical system condition information LS and for each specific area R. The wavefront state $\theta$ represents a wavefront state of the wavefront control device 3 for each pixel. The imaging performance evaluation value Y is calculated by multiplying a value of the image signal IS by a weighting factor WY for each area R of the image P. In other words, the wavefront state table T is generated on the basis of the image signal IS.

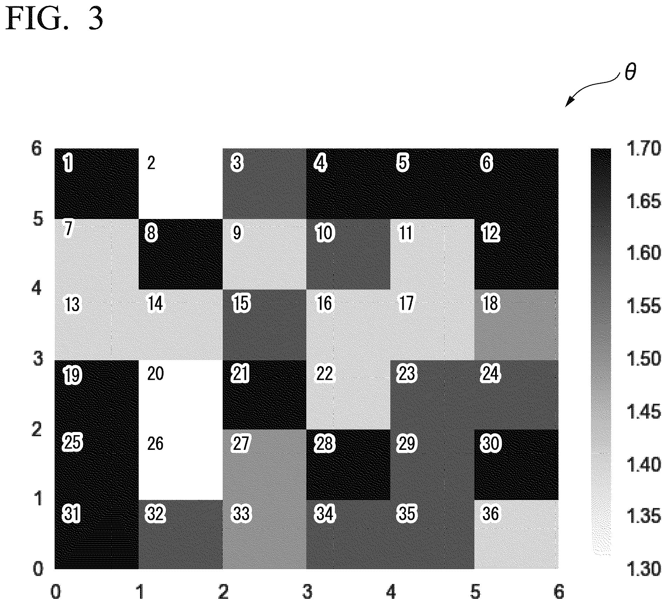

Here, the wavefront state of the wavefront control device 3 will be described with reference to Fig. 3.

Fig. 3 is a diagram illustrating an example of a wavefront state of the wavefront control device 3 according to this embodiment. In Fig. 3, a delay of a phase of a wavefront is represented using white and black shading for every 36 pixels.

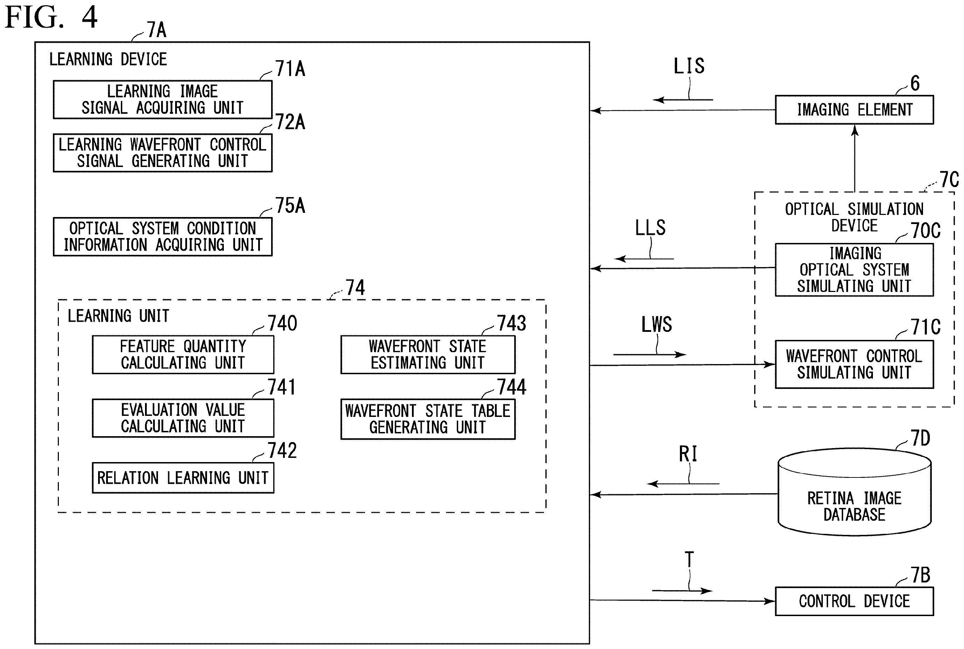

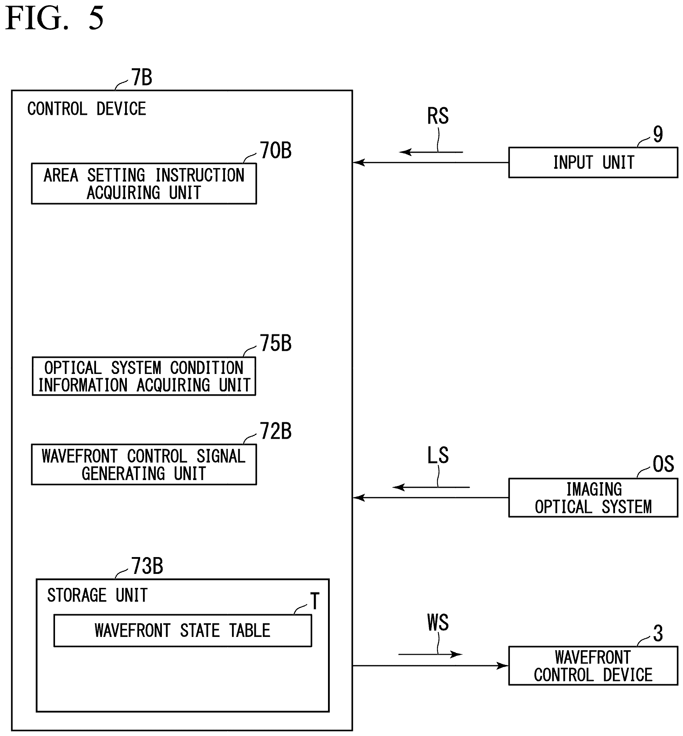

Next, details of the configuration of the computer 7 will be described with reference to Figs. 4 and 5.

Fig. 4 is a diagram illustrating an example of the configuration of the learning device 7A according to this embodiment. The learning device 7A generates a wavefront state table T on the basis of an optical simulation using the optical simulation device 7C and supplies the wavefront state table T to the control device 7B. In order to generate a wavefront state table T, the learning device 7A may use retina image data RI stored in the retina image database 7D.

In the process of generating a wavefront state table T, the learning device 7A uses the optical simulation device 7C and the retina image database 7D.

The learning device 7A, the optical simulation device 7C, and the retina image database 7D may be configured to be included in an external computer, which is independent from the computer 7, instead of being included in the computer 7.

Hereinafter, the process of generating a wavefront state table T will be referred to as a learning process.

The learning device 7A includes a learning image signal acquiring unit 71A, a learning wavefront control signal generating unit 72A, a learning unit 74, and an optical system condition acquiring unit 75A.

The learning image signal acquiring unit 71A acquires a learning image signal LIS supplied from the imaging element 6. The learning image signal LIS is a signal that represents a value of each pixel of an image P generated by the imaging element 6 in the learning process.

The learning wavefront control signal generating unit 72A supplies a learning wavefront control signal LWS to the optical simulation device 7C. The learning wavefront control signal LWS is a signal that is used for generating a wavefront state $\theta$ in an optical simulation in the learning process.

The optical system condition acquiring unit 75A acquires learning optical system condition information LLS from the optical simulation device 7C. The learning optical system condition information LLS is information that represents optical system conditions A used for an optical simulation in the learning process. The optical system conditions A, for example, are designated using a zoom magnification of the imaging optical system OS, types of the objective lens system 2 and the imaging lens system 5, the size of the diaphragm 4, a type of subject 1, and the like. In addition, the optical system conditions A are also designated depending on whether the imaging system S (an optical device D), which is a microscope for ophthalmology, is for observation of an anterior eye part or is for observation of a posterior eye part.

The learning unit 74 executes machine learning for generating a wavefront state table T.

The learning unit 74 includes a feature quantity calculating unit 740, an evaluation value calculating unit 741, a relation learning unit 742, a wavefront state estimating unit 743, and a wavefront state table generating unit 744.

The feature quantity calculating unit 740 calculates a feature quantity of a retina image from the retina image data RI stored in the retina image database 7D. For example, the feature quantity of the retina image is a feature quantity of a peripheral part or a center part of a retina.

The evaluation value calculating unit 741 calculates an imaging performance evaluation value Y on the basis of the learning image signal LIS and the weighting factor WY supplied from the imaging element 6. The evaluation value calculating unit 741 may change the value of the weighting factor WY on the basis of the feature quantity calculated by the feature quantity calculating unit 740.

The relation learning unit 742 learns a relation between the wavefront state θ and the imaging performance evaluation value Y for a specific area R. For example, the relation learning unit 742 uses machine learning that uses an algorithm such as a support vector regression (SVR) or the like.

The wavefront state estimating unit 743 estimates a wavefront state θ for which the imaging performance evaluation value Y is maximized for a specific area R on the basis of a learning result acquired by the relation learning unit 742.

The wavefront state table generating unit 744 generates a wavefront state table T on the basis of an estimation result acquired by the wavefront state estimating unit 743.

The optical simulation device 7C includes an imaging optical system simulating unit 70C and a wavefront control simulating unit 71C.

The imaging optical system simulating unit 70C executes an optical simulation of the imaging optical system OS on the basis of optical system data DT. The optical system data DT is a set of a wavefront state θ and optical system conditions A for an optical simulation. The imaging optical system simulating unit 70C maintains predetermined conditions corresponding to a predetermined number in advance as the optical system conditions A.

The imaging optical system simulating unit 70C generates learning optical system condition information LLS on the basis of the optical system conditions A.

The wavefront control simulating unit 71C generates a wavefront state θ for an optical simulation on the basis of the learning wavefront control signal LWS supplied from the learning device 7A and supplies the generated wavefront state θ to the imaging optical system simulating unit 70C.

Next, the configuration of the control device 7B will be described with reference to Fig. 5.

Fig. 5 is a diagram illustrating an example of the configuration of the control device 7B according to this embodiment. The control device 7B includes an area setting information acquiring unit 70B, an optical system condition information acquiring unit 75B, a wavefront control signal generating unit 72B, and a storage unit 73B.

The area setting information acquiring unit 70B acquires an area setting instruction RS supplied from the input unit 9. The area setting instruction RS is information that represents a specific area RC designated by a user of the imaging system S through the input unit 9.

The optical system condition information acquiring unit 75B acquires optical system condition information LS from the imaging optical system OS. The optical system condition information LS is information that represents optical system conditions A of the imaging optical system OS.

The wavefront control signal generating unit 72B supplies a wavefront control signal WS to the wavefront control device 3. The wavefront control signal WS is a signal that represents a wavefront state θ.

A wavefront state table T is stored in the storage unit 73B. As described above, the wavefront state table T is generated by the learning device 7A in advance in the learning process.

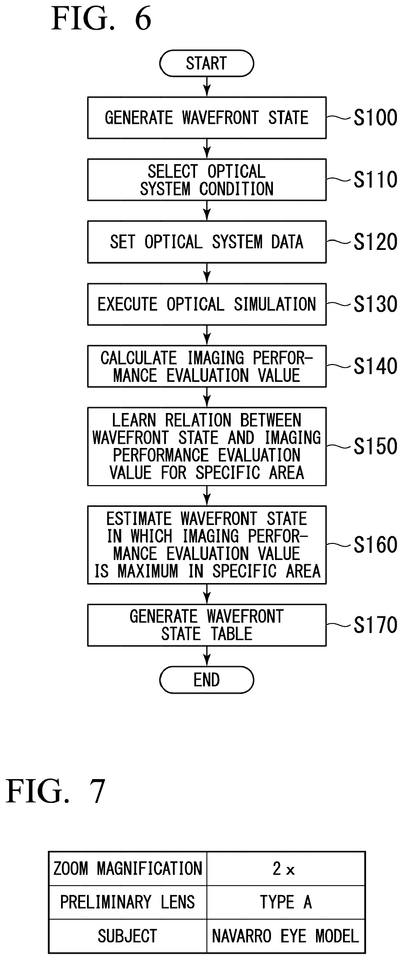

Next, a learning process using the learning device 7A and the optical simulation device 7C will be described with reference to Fig. 6.

Fig. 6 is a diagram illustrating an example of the learning process according to this embodiment. The learning process illustrated in Fig. 6 is executed in advance before control of the wavefront control device 3 using the control device 7B is executed.

Step S100: The wavefront control simulating unit 71C generates a wavefront state θ that is used for an optical simulation. Here, the wavefront control simulating unit 71C generates a wavefront state θ on the basis of the learning wavefront control signal LWS supplied from the learning wavefront control signal generating unit 72A. The wavefront control simulating unit 71C supplies the generated wavefront state θ to the imaging optical system simulating unit 70C.

The learning wavefront control signal generating unit 72A generates a learning wavefront control signal LWS as a signal representing one of N wavefront states θ. The learning wavefront control signal generating unit 72A randomly generates the N wavefront states θ. A pattern in which the learning wavefront control signal generating unit 72A randomly generates a wavefront state θ may have center symmetry or line symmetry dividing an element into two parts or may be a pattern that is represented using a Zernike approximate polynomial expression.

A set of the N wavefront states θ is represented using Equation (1).

[Math 1]

$$\Theta = \{\theta_i\}_{i=1}^{N} \cdots (1)$$

Step S110: The imaging optical system simulating unit 70C selects an optical system condition A used for the optical system data DT from among maintained predetermined conditions. The imaging optical system simulating unit 70C selects one or more optical system conditions A from among the maintained predetermined conditions.

Here, an example of the optical system conditions A will be described with reference to Fig. 7.

Fig. 7 is a diagram illustrating an example of optical system conditions A according to this embodiment. In the example of the optical system conditions A illustrated in Fig. 7, "2 times" is selected as a zoom magnification, "type A" is selected as a preliminary lens, and "Navarro eye model" is selected as a subject 1.

The description of the learning process will be continued with reference back to Fig. 6.

Step S120: The imaging optical system simulating unit 70C sets optical system data DT used for an optical simulation. Here, the imaging optical system simulating unit 70C generates M pieces of optical system data DT using each of the N wavefront states θ generated by the wavefront control simulating unit 71C in Step S100 and the optical system condition A selected in Step S110 as a set. M is equal to a product of the number of optical system conditions A selected in Step S110 and N.

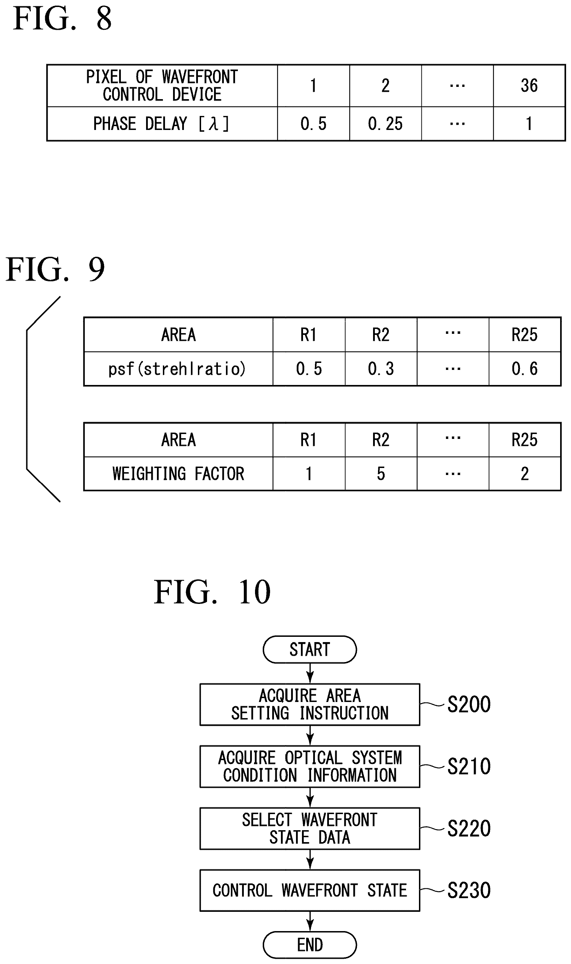

Here, an example of the wavefront state θ will be described with reference to Fig. 8.

Fig. 8 is a diagram illustrating an example of the wavefront state θ according to this embodiment. In the example illustrated in Fig. 8, for each pixel of the wavefront control device 3, a value of a delay of a phase of a wavefront in a pupil of the imaging optical system OS is represented.

The description of the learning process will be continued with reference back to Fig. 6.

Step S130: The imaging optical system simulating unit 70C executes an optical simulation. The imaging optical system simulating unit 70C executes an optical simulation in association with each of the M pieces of optical system data DT. In other words, the imaging optical system simulating unit 70C executes an optical simulation for the N wavefront states θ that have been randomly generated in Step S100 for each optical system condition A.

For example, the imaging optical system simulating unit 70C executes an optical simulation using known optical design analysis software.

The imaging optical system simulating unit 70C generates an image of a subject 1 using an optical simulation. The imaging element 6 generates an image P from the generated image of the subject 1. As images P, there are images PI to PN in association with the N wavefront states θ for each optical system condition A.

The imaging element 6 includes values of pixels of each of the images PI to PN for each optical system condition A in the learning image signal LIS and outputs them to the learning device 7A.

Hereinafter, one of the image PI to the image PN may be representatively referred to as an image Pi.

Step S140: The evaluation value calculating unit 741 calculates an imaging performance evaluation value Y on the basis of the learning image signal LIS supplied from the imaging element 6 and the weighting factor WY.

The evaluation value calculating unit 741 calculates an imaging performance PY for each area R of an image Pi on the basis of values of pixels of the image Pi included in the learning image signal LIS. Here, the imaging performance PY for each area R of the image P is, for example, a value of a point spread function (PSF). In addition, the imaging performance PY may be a contrast value, a line spread function (LSF), or a modulation transfer function (MTF).

The evaluation value calculating unit 741, for each area R, sets the weighting factor WY such that the imaging performance evaluation value Y of the area R is relatively larger than the imaging performance evaluation value Y of an area other than the area R in an area R. The evaluation value calculating unit 741 calculates the imaging performance evaluation value Y by multiplying the imaging performance PY of each area R by the weighting factor WY set for each area R. Here, the imaging performance PY of the area R is higher as the imaging performance evaluation value Y is larger.

The evaluation value calculating unit 741 repeats the process of Step S140 for the image PI to the image PN for each optical system condition A.

Here, a specific example of the imaging performance PY and the weighting factor WY will be described with reference to Fig. 9.

Fig. 9 is a diagram illustrating an example of the imaging performance PY and the weighting factor WY according to this embodiment. In Fig. 9, a value of the PSF is illustrated as the imaging performance PY for each of the area R1 to the area R25. In addition, in Fig. 9, values of the weighting factor WY1 to the weighting factor WY25 are respectively illustrated for the area R1 to the area R25.

In the example illustrated in Fig. 9, the imaging performance evaluation value Y1 for the area R1 is calculated as 0.5, the imaging performance evaluation value Y2 for the area R2 is calculated as 1.5, and the imaging performance evaluation value Y25 for the area R25 is calculated as 1.2.

The description of the learning process will be continued with reference back to Fig. 6.

A set of imaging performance evaluation values Y is represented using Equation (2).

[Math 2]

$$Y = \{y_i\}_{I=1}^{N} \cdots (2)$$

The evaluation value calculating unit 741 may change the value of the weighting factor WY on the basis of a feature quantity calculated by the feature quantity calculating unit 740. For example, the evaluation value calculating unit 741 may change values of the weighting factor WY12, the weighting factor WY13, the weighting factor WY17, and the weighting factor WY18 respectively corresponding to the area R12, the area R13, the area R17, and the area R18 of the image P illustrated in Fig. 2 to values that are 1.2 times the original values on the basis of a feature quantity of the center part of the retina calculated by the feature quantity calculating unit 740.

Step S150: The relation learning unit 742 learns a relation between the wavefront state $\theta$ and the imaging performance evaluation value Y for a specific area R. Here, for example, the relation learning unit 742 uses machine learning using an SVR. For example, the relation learning unit 742 learns a relation between the wavefront state $\theta$ and the imaging performance evaluation value Y for a specific area R on the basis of Equation (3). In Equation (3), "Loss" represents an appropriate distance between the wavefront state $\theta$ and the imaging performance evaluation value Y, and, for example, a square distance or the like may be used.

[Math 3]

$$f_{\sigma}^{\cdot} = arg \; \min_{f} Loss \left( \{y_{\sigma,i}\}_{i=1}^{N}, \{f_{\sigma}(\theta_i)\}_{i=1}^{N} \right) \cdots (3)$$

Step S160: The wavefront state estimating unit 743 estimates a wavefront state $\theta$ having a maximum imaging performance evaluation value Y for the specific area R. Here, the wavefront state estimating unit 743 estimates a wavefront state $\theta$ using machine learning that uses the SVR. For example, the wavefront state estimating unit 743 estimates a wavefront state $\theta$ for which a relation f between the learned wavefront state $\theta$ and the imaging performance evaluation value Y is maximized on the basis of Equation (4).

[Math 4]

$$\theta_{\sigma}^{\cdot} = arg \; \max_{\theta} \; f_{\sigma}^{\cdot} \cdots (4)$$

Although a case in which the SVR is used as an algorithm of machine learning in Step S150 and Step S160 has been described in this embodiment, the algorithm is not limited thereto.

In Step S150, in order to learn the relation between the wavefront state $\theta$ and the imaging performance evaluation value Y, the relation learning unit 742 may use any one of a parametric regression and a non-parametric regression. The SVR is an example of an algorithm of a nonlinear nonparametric regression.

In Step S160, in order to estimate the wavefront state $\theta$, the wavefront state estimating unit 743 may use a grid search or a Markov chain Monte Carlo method (MCMC).

In addition, in Step S150 and Step S160, Bayesian optimization using a Gaussian process regression may be used. The efficiency of a solution search using Bayesian optimization that uses the Gauss process regression may be improved more than that of this embodiment. The efficiency of the solution search is accuracy of the solution or a convergence speed of learning.

In addition, the wavefront state $\theta$ of the wavefront control device 3 may be represented using a Zernike coefficient instead of being represented in units of pixels. In a case in which the wavefront state $\theta$ of the wavefront control device 3 is represented using the Zernike coefficient, the efficiency of the solution search may be improved more than that according to this embodiment.

Step S170: The wavefront state table generating unit 744 generates a wavefront state table T on the basis of an estimation result acquired by the wavefront state estimating unit 743 in Step S160. The wavefront state table generating unit 744 supplies the generated wavefront state table T to the control device 7B.

Next, a control process for the wavefront control device 3 using the control device 7B will be described with reference to Fig. 10.

Fig. 10 is a diagram illustrating an example of the control process for the wavefront control device 3 using the control device 7B according to this embodiment. The control process illustrated in Fig. 10 is executed after the learning process illustrated in Fig. 6 is executed.

Step S200: The area setting information acquiring unit 70B acquires an area setting instruction RS supplied from the input unit 9. The area setting information acquiring unit 70B supplies the acquired area setting instruction RS to the wavefront control signal generating unit 72B.

Step S210: The optical system condition information acquiring unit 75B acquires optical system condition information LS from the imaging optical system OS. The optical system condition information acquiring unit 75B supplies the acquired optical system condition information LS to the wavefront control signal generating unit 72B.

Step S220: The wavefront control signal generating unit 72B selects the wavefront state table T. Here, the wavefront control signal generating unit 72B sets a specific area RC on the basis of the area setting instruction RS supplied from the area setting information acquiring unit 70B. In addition, the wavefront control signal generating unit 72B sets an optical system condition A on the basis of the optical system condition information LS supplied from the optical system condition information acquiring unit 75B. The wavefront control signal generating unit 72B selects a wavefront state table T corresponding to the set optical system condition A and the set specific area RC from among wavefront state tables T stored in the storage unit 73B.

Here, as described above, the area setting instruction RS is information that represents a specific area RC designated by a user of the imaging system S through the input unit 9. In other words, the specific area RC is set on the basis of an input accepted by the input unit 9.

Step S230: The wavefront control signal generating unit 72B controls the wavefront state θ of the wavefront control device 3. Here, the wavefront control signal generating unit 72B determines the wavefront state θ on the basis of the wavefront state table T selected in Step S220. The wavefront control signal generating unit 72B generates a wavefront control signal WS that represents the determined wavefront state θ. The wavefront control signal generating unit 72B supplies the generated wavefront control signal WS to the wavefront control device 3, thereby controlling the wavefront state θ of the wavefront control device 3.

The wavefront state table T selected in Step S220 corresponds to the specific area RC of the image P acquired from the image signal IS. A wavefront of an area corresponding to the specific area RC is included in the wavefront state θ of the wavefront control device 3 controlled by the wavefront control signal generating unit 72B. In other words, the wavefront control signal generating unit 72B performs control of the wavefront control device 3 such that a phase of the wavefront of an area in the pupil of the imaging optical system OS corresponding to the specific area RC of the image P acquired from the image signal IS is changed.

As described above, the wavefront state table T is generated by the learning device 7A on the basis of an image signal IS output by the imaging element 6 in the learning process. The wavefront control signal generating unit 72B controls the wavefront control device 3 on the basis of the wavefront state table T. In other words, the control device 7B controls the wavefront control device 3 on the basis of an image signal IS output by the imaging element 6.

As above, the control device 7B ends the control process performed by the wavefront control device 3.

(Modified example of first embodiment)

In the first embodiment described above, a case in which an optical simulation is used for generating a wavefront state table T has been described. Here, as a modified example of the first embodiment, a case in which imaging using an actual imaging system S (an optical device D) is used for generating a wavefront state table T instead of the optical simulation will be described.

A learning device according to this modified example will be referred to as a learning device 7Aa.

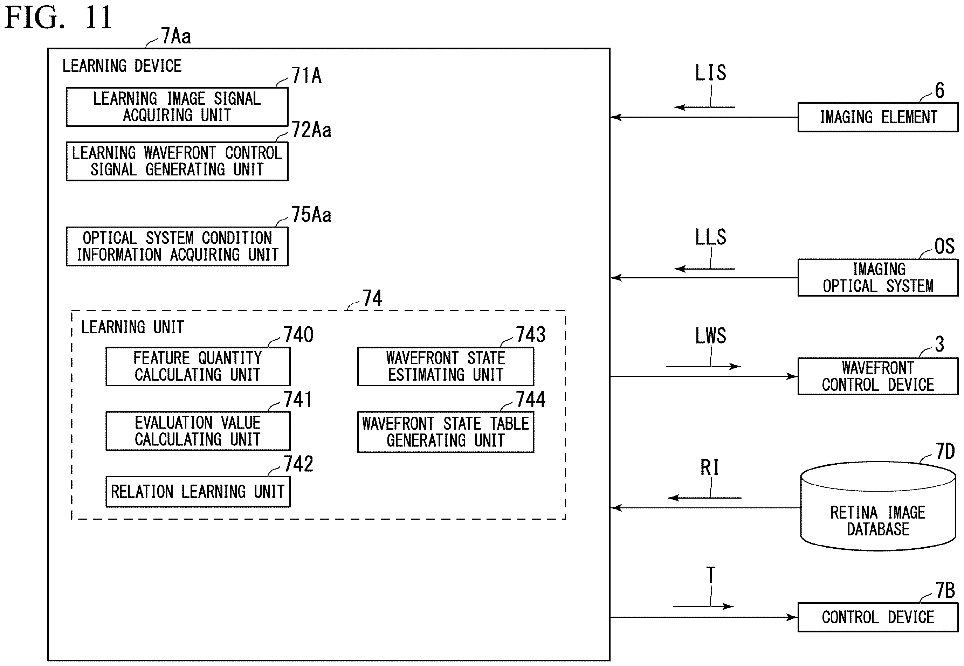

Fig. 11 is a diagram illustrating an example of the configuration of a learning device 7A according to this embodiment. When the learning device 7Aa (Fig. 11) according to this modified example is compared with the learning device 7A (Fig. 4) according to the first embodiment, there is a difference in that it includes a learning wavefront control signal generating unit 72Aa, an optical system condition acquiring unit 75Aa, and an imaging optical system OS and a wavefront control device 3 in place of the optical simulation device 7C. Here, the functions of the constituent elements (the learning image signal acquiring unit 71A and the learning unit 74) of the learning device 7A are the same as those according to the first embodiment. Description of the functions that are the same as those of the first embodiment will be omitted, and, parts different from the first embodiment will be focused on in a description in the modified example.

The learning wavefront control signal generating unit 72A supplies a learning wavefront control signal LWS to a wavefront control device 3.

The optical system condition acquiring unit 75A acquires learning optical system condition information LLS from the imaging optical system OS.

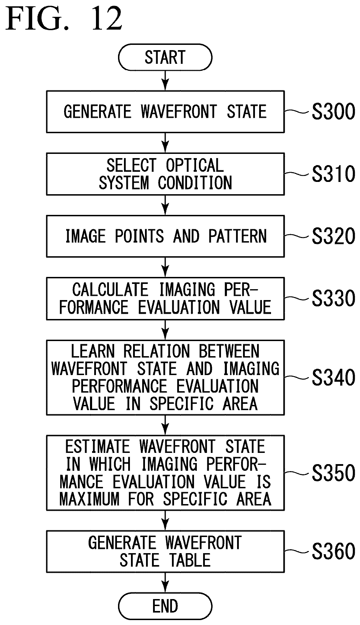

Here, a learning process according to this modified example will be described with reference to Fig. 12.

Fig. 12 is a diagram illustrating an example of a learning process according to this modified example. Processes of Steps S300, S310, S330, S340, S350, and S360 are similar to the processes of Steps S100, S110, S140, S150, S160, and S170 illustrated in Fig. 6, and thus description thereof will be omitted.

Step S320: An imaging element 6 images points and a pattern as a subject 1. Here, for example, a paper sheet on which points and a pattern are printed is arranged as the subject 1 in an imaging system S. As the pattern, for example, a resolution chart may be used. The imaging element 6 images points and a pattern in association with each of M pieces of optical system data DT. In other words, the imaging element 6 images points and a pattern for N wavefront states that have been randomly generated in Step S300.

The imaging element 6 generates, for each optical system condition A, an image PI to an image PN from an image of the points and the pattern that have been imaged. The imaging element 6 outputs values of pixels of the image P1 to the image PN for each optical condition A to the learning device 7A with being included in a learning image signal LIS.

In this modified example, in generating a wavefront state table T, imaging using an actual imaging system S (an optical device D) is used, and thus, a manufacture tolerance of the imaging system S (the optical device D) is reflected on the wavefront state table T. Therefore, the wavefront state θ of the wavefront control device 3 can be controlled without being affected by the manufacture tolerance.

As described above, the optical device D according to this embodiment includes an imaging optical system OS, an imaging element 6, a spatial modulation element (the wavefront control device 3 in this example), and a control unit (the control device 7B in this example).

The imaging optical system OS forms an image of a subject 1.

The imaging element 6 outputs an image signal IS of the image of the subject 1 formed by the imaging optical system OS.

The wavefront control device 3 changes a phase of a wavefront in the pupil of the imaging optical system OS.

The control unit (the control device 7B in this example) controls the wavefront control device 3 on the basis of the image signal IS output by the imaging element 6.

According to this configuration, the optical device D according to this embodiment can control the wavefront control device 3 on the basis of the image signal IS, and thus the imaging performance of the optical device can be improved without using a wavefront measuring device.

In addition, in the optical device D according to this embodiment, the wavefront control device 3 is disposed at a position conjugate with the pupil of the imaging optical system OS.

By employing such a configuration, the optical device D according to this embodiment can control the phase of the wavefront in the pupil of the imaging optical system OS.

In addition, in the optical device D according to this embodiment, the control unit (the control device 7B in this example) performs control of the wavefront control device 3 such that the phase of the wavefront of an area in the pupil corresponding to a specific area RC of an image P acquired from the image signal IS is changed.

By employing such a configuration, the optical device D according to this embodiment can change the phase of the wavefront of the area corresponding to the specific area RC of the image P and thus can improve the imaging performance of the specific area RC of the image P

In addition, the optical device D according to this embodiment has an input unit 9 that accepts an input from a user, and the specific area RC is set on the basis of the input accepted by the input unit 9.

In the optical device D according to this embodiment, a user can set a specific area RC, and thus the user can improve the imaging performance of an image angle with which the user desires to gaze in the image P.

(Second embodiment)

Hereinafter, a second embodiment of the present invention will be described in detail with reference to the drawings.

In the first embodiment described above, a case in which the imaging system (the optical device) controls the wavefront control device on the basis of a learning result acquired by learning the relationship between an image signal and a phase of a wavefront in the pupil of the imaging optical system in advance has been described. In this embodiment, a case in which an imaging system (an optical device) immediately learns the relationship between an image signal and a phase of the wavefront in the pupil of the imaging optical system during use, and a learning result is updated will be described.

An imaging system according to this embodiment will be referred to as an imaging system Sb, and an optical device will be referred to as an optical device Db.

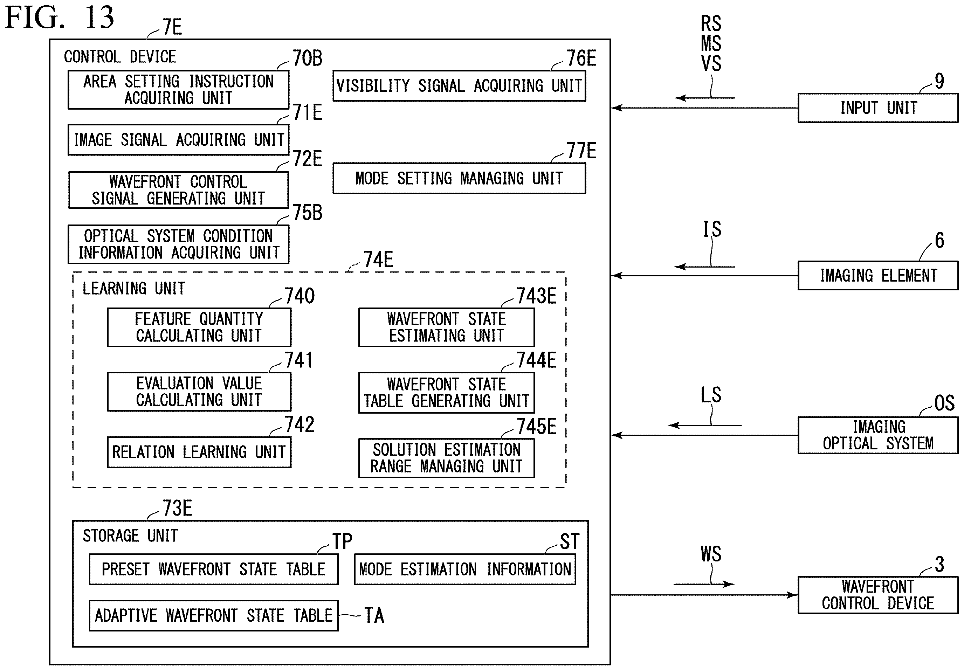

Fig. 13 is a diagram illustrating the configuration of a control device 7E according to this embodiment. When the control device 7E (Fig. 13) according to this embodiment is compared with the control device 7B (Fig. 5) according to the first embodiment, an image signal acquiring unit 71E, a wavefront control signal generating unit 72E, a learning unit 74E, a storage unit 73E, a visibility signal acquiring unit 76E, and a mode setting managing unit 77E are different from the control device 7B. Here, functions of other constituent elements (the area setting information acquiring unit 70B and the optical system condition information acquiring unit 75B) are the same as those according to the first embodiment. A description of the same functions as those according to the first embodiment will be omitted, and, in the second embodiment, parts different from the first embodiment will be focused in description.

The image signal acquiring unit 71E acquires an image signal IS of an operation image OP supplied from an imaging element 6. The image signal IS a signal that represents a value of each pixel of the operation image OP generated by the imaging element 6.

The wavefront control signal generating unit 72E selects one of a preset wavefront state table TP and an adaptive wavefront state table TA as a wavefront state table T in accordance with mode setting information ST stored in the storage unit 73E.

The visibility signal acquiring unit 76E acquires visibility information VS supplied from an input unit 9. Here, the visibility information VS is a signal that represents a result of determination of visibility of an operation image OP displayed in a display unit 8 of a user of an imaging system Sb (an optical device Db). As results of determination of visibility of an operation image OP, there are "visible" and "invisible". "Visible" is a result of determination in a case in which the resolution of an area of an operation image OP, at which a user gazes, displayed in the display unit 8 is sufficient. "Invisible" is a result of determination in a case in which the resolution of an area of an operation image OP, at which a user gazes, displayed in the display unit 8 is insufficient.

The mode setting information ST is information that represents a setting of whether the preset wavefront state table TP or the adaptive wavefront state table TA is used as the wavefront state table T. As values of the mode setting information ST, there are "preset" representing that the preset wavefront state table TP is used and "adaptive" representing that the adaptive wavefront state table TA is used.

The preset wavefront state table TP is a table that is the same as the wavefront state table T (Fig. 5), is generated through learning in advance, and is stored in the storage unit 73E. The adaptive wavefront state table TA is generated on the basis of an operation image OP of the retina of a patient imaged using the imaging system Sb (the optical device Db).

The learning unit 74E learns the relationship between an image signal and a phase of a wavefront in the pupil of the imaging optical system during use of the imaging system Sb (the optical device Db). The learning unit 74E generates an adaptive wavefront state table TA as a result of learning and causes the storage unit 73E to store the generated adaptive wavefront state table TA.

Here, when the learning unit 74E (Fig. 13) is compared with the learning unit 74 (Fig. 4) of the learning device 7A, a wavefront state estimating unit 743E, a wavefront state table generating unit 744E, and a solution estimation range managing unit 745E are different from the learning unit 74. The functions of the other constituent elements (the feature quantity calculating unit 740, the evaluation value calculating unit 741, and the relation learning unit 742) are the same as those of the learning unit 74 (Fig. 4).

The wavefront state estimating unit 743E has the function of the wavefront state estimating unit 743 and estimates a wavefront state $\theta$ for which the imaging performance evaluation value Y is maximized for a specific area R within a range set by the solution estimation range managing unit 745E on the basis of a learning result acquired by the relation learning unit 742.

The wavefront state table generating unit 744E has the function of the wavefront state table generating unit 744 and generates an adaptive wavefront state table TA on the basis of an estimation result acquired by the wavefront state estimating unit 743E.

The solution estimation range managing unit 745E sets a range of the wavefront state $\theta$ estimated by the wavefront state estimating unit 743E.

The preset wavefront state table TP, the adaptive wavefront state table TA, and the mode setting information ST are stored in the storage unit 73E.

The mode setting managing unit 77E sets the mode setting information ST stored in the storage unit 73E in accordance with a setting represented by the mode setting instruction MS supplied from the input unit 9.

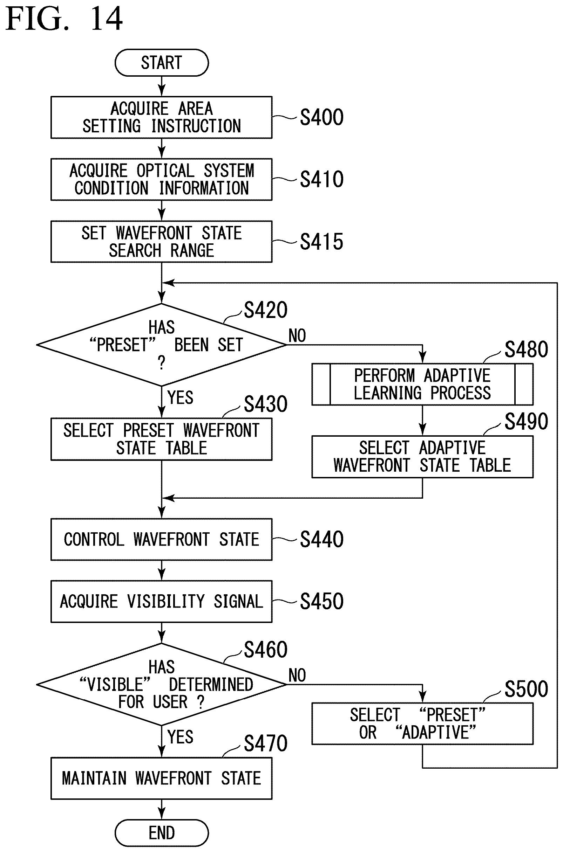

Fig. 14 is a diagram illustrating an example of a control process for the wavefront control device 3 using the control device 7E according to this embodiment. Processes of Step S400 and Step S410 are similar to the processes of Step S200 and Step S210 illustrated in Fig. 10, and thus description thereof will be omitted.

Step S415: The solution estimation range managing unit 745E sets a range $\Omega$ of the solution of the wavefront state $\theta$ estimated by the wavefront state estimating unit 743E. Here, the solution estimation range managing unit 745E sets the range $\Omega$ of the solution of the wavefront state $\theta 0$ on the basis of a specific area RC represented by an area setting instruction RS acquired in Step S400, an optical system condition $\Lambda 0$ acquired in Step S410, and the preset wavefront state table TP.

The range $\Omega$ set by the solution estimation range managing unit 745E is represented as in Equation (5). $\Delta$ represented in Equation (5) is a quantity for allowing the preset wavefront state table TP to have a width and is set in advance.

[Math 5]

$$\Omega = T(\sigma_0, \Lambda_0) + \Delta \cdots (5)$$

Step S420: The wavefront control signal generating unit 72E determines whether or not the mode setting is set to "preset". Here, the wavefront control signal generating unit 72E reads the mode setting information ST stored in the storage unit 73E and performs determination.

In a case in which it is determined that the mode setting is set to "preset" (Step S420: Yes), the wavefront control signal generating unit 72E executes the process of Step S430. On the other hand, in a case in which it is determined that the mode setting is not set to "preset" (Step S420: No), the wavefront control signal generating unit 72E executes the process of Step S470.

Step S430: The wavefront control signal generating unit 72E selects the preset wavefront state table TP as a wavefront state table T.

Step S440: The wavefront control signal generating unit 72E controls the wavefront state $\theta$ of the wavefront control device 3. Here, the wavefront control signal generating unit 72E determines a wavefront state $\theta$ on the basis of the wavefront state table T selected in Step S430 or Step S490. The wavefront control signal generating unit 72E generates a wavefront control signal WS that represents the determined wavefront state $\theta$. The wavefront control signal generating unit 72E supplies the generated wavefront control signal WS to the wavefront control device 3, thereby controlling the wavefront state $\theta$ of the wavefront control device 3.

The preset wavefront state table TP or the adaptive wavefront state table TA is a learning result acquired by the learning unit 74E. The preset wavefront state table TP is a learning result acquired by learning the relationship between an image signal IS and a phase of the wavefront in the pupil of the imaging optical system OS in advance. The adaptive wavefront state table TA immediately learns the relationship between an image signal IS of an operation image OP and a phase of the wavefront in the pupil of the imaging optical system OS during an operation and is generated by changing the preset wavefront state table TP.

In other words, the control device 7E controls the wavefront control device 3 on the basis of a learning result acquired by the learning unit 74E and an image signal IS output by the imaging element 6. Here, the learning unit 74E has learned the relationship between an image signal IS and a phase of the wavefront in the pupil of the imaging optical system OS in advance.

Step S450: The visibility signal acquiring unit 76E acquires visibility information VS supplied from the input unit 9. The visibility signal acquiring unit 76E supplies the acquired visibility information VS to the wavefront control signal generating unit 72E.

Step S460: The wavefront control signal generating unit 72E determines whether or not visibility of an operation image OP displayed in the display unit 8 for a user of the imaging system Sb (the optical device Db) has been determined to be "visible". In a case in which the visibility information VS acquired in Step S450 represents "visible", the wavefront control signal generating unit 72E determines that the visibility of the operation image OP for the user has been determined to be "visible". On the other hand, in a case in which the visibility information VS acquired in Step S450 represents "invisible", the wavefront control signal generating unit 72E determines that the visibility of the operation image OP for the user has been determined to be "invisible".

In a case in which it is determined that the visibility of the operation image OP for the user has been determined to be "visible" (Step S460: Yes), the wavefront control signal generating unit 72E executes the process of Step S470. On the other hand, in a case in which the wavefront control signal generating unit 72E determines that the visibility of the operation image OP for the user is not "visible" (Step S460: No), the control device 7E executes the process of Step S500.

Step S470: The wavefront control signal generating unit 72E maintains the wavefront state $\theta$ of the wavefront control device 3. In other words, in a case in which it is determined that the visibility of the operation image OP for the user has been determined to be "visible" in Step S460, the wavefront control signal generating unit 72E maintains the wavefront state $\theta$ of the wavefront control device 3. Thus, the control device 7B fixes a state in which the wavefront control device 3 is controlled on the basis of a predetermined condition.

Step S480: The learning unit 74E executes an adaptive learning process.

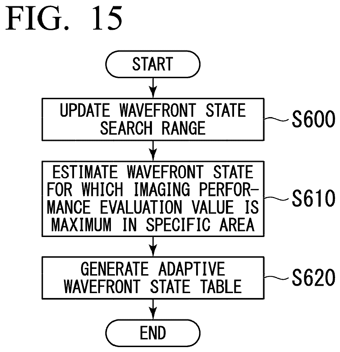

Here, the adaptive learning process performed by the learning unit 74E will be described with reference to Fig. 15.

Fig. 15 is a diagram illustrating an example of the adaptive learning process according to this embodiment. The adaptive learning process illustrated in Fig. 15 corresponds to Step S480 illustrated in Fig. 14.

Step S600: The solution estimation range managing unit 745E updates the range of the solution of the wavefront state $\theta$ estimated by the wavefront state estimating unit 743E in the adaptive learning process of Step S480 in the range $\Omega$ set in Step S415. Here, the solution estimation range managing unit 745E updates the range of the solution of the wavefront state $\theta$ estimated by the wavefront state estimating unit 743E by shifting the range from the wavefront state $\theta$ selected on the basis of the preset wavefront state table TP by an amount of change $\delta$. For example, the solution estimation range managing unit 745E updates the range of the solution of the wavefront state $\theta$ estimated by the wavefront state estimating unit 743E as in Equation (6). The amount of change $\delta$ is set in advance.

[Math 6]

$$\Omega \leftarrow \theta_0^{\cdot} + \delta \cdots (6)$$

The solution estimation range managing unit 745E updates the range of the solution of the wavefront state $\theta$ estimated by the wavefront state estimating unit 743E with the range $\Omega$ set in Step S415.

Step S610: The wavefront state estimating unit 743E estimates a wavefront state $\theta$ for which the imaging performance evaluation value Y is maximized for a specific area RC in the updated range of the solution of the wavefront state θ in Step S600 as in Equation (7).
[Math 7]

$$\theta_0^{\cdot} = \max_{|\theta^{\cdot}| \leq \Omega} Y(\theta^{\cdot}; \sigma_0, \Lambda_0, T) \cdots (7)$$

Step S612: The wavefront state table generating unit 744E generates an adaptive wavefront state table TA on the basis of an estimation result acquired in Step S610. The wavefront state table generating unit 744E updates the adaptive wavefront state table TA stored in the storage unit 73E using the generated adaptive wavefront state table TA.

The description of the control process of the wavefront control device 3 using the control device 7E will be continued with reference to Fig. 14.

Step S490: The wavefront control signal generating unit 72E selects the adaptive wavefront state table TA as a wavefront state table T.

Step S500: The mode setting managing unit 77E selects "preset" or "adaptive" as the mode setting information ST. Here, the mode setting managing unit 77E acquires a mode setting instruction MS supplied from the input unit 9. The mode setting managing unit 77E sets the mode setting information ST stored in the storage unit 73E in accordance with a setting represented by the acquired mode setting instruction MS. Here, the mode setting instruction MS represents a setting of one of "preset" and "adaptive".

In the processes of Fig. 14 and Fig. 15 described above, in a case in which the visibility of the operation image OP displayed in the display unit 8 is not determined to be "visible" in Step S460, the wavefront control signal generating unit 72E may perform control of the wavefront control device 3 such that the wavefront state θ of the wavefront control device 3 is uniform.

As described above, in the optical device Db according to this embodiment, a control unit (the control device 7E in this example) fixes the state in which the wavefront control device 3 is controlled on the basis of a predetermined condition (the determination condition of Step S460).

By employing such a configuration, the optical device Db according to this embodiment can fix the state in which the wavefront control device 3 is controlled, and thus, by controlling the wavefront state θ of the wavefront control device 3 once, and thus an operation image OP having imaging performance desired by a user can be continuously acquired. In the optical device Db according to this embodiment, differently from a case in which an image is processed to have high definition through image processing, a process for each frame is not required. In the optical device Db according to this embodiment, in a case in which an operation image OP is immediately observed as a moving image, a delay according to the process of the optical device does not occur.

Here, in an ophthalmic surgery, the resolution of a center part or a peripheral part of an image of an eye of a patient is low, and thus there are problems of a case in which a disease is not found and a case in which an operation is difficult to perform. For example, an area such as a center part or a peripheral part at which a user desires to gaze is unclearly displayed in accordance with obstacles such as a phase object and the like.

In recent years, in accordance with improvement of image processing technologies, an image having higher definition than that of an image acquired by an imaging element can be observed. However, in image processing technologies, each frame needs to be processed or arithmetically operated, and, thus, in a case in which a moving image is observed in real time during an ophthalmic surgery, a delay occurs when the observation is performed. In addition, in a case in which a user gazes at a specific area using an electronic zoom, an image projected to a sensor does not change, and thus, the resolution of an observed image is lower than that of an optical zoom.

In the optical device Db according to this embodiment, not only display can be enlarged, but also the wavefront can be corrected, and thus an image having higher definition than that acquired using an electronic zoom that is a conventional technology can be acquired.

On the other hand, it may be considered to improve resolution of an image of an eye of a patient by changing the position of an imaging device such as a microscope or the like. However, the position of the microscope cannot be easily changed from the point of view of focusing and pupil alignment. In addition, it is difficult to understand an operation of the microscope that enables acquisition of a desired image.

The optical device Db according to this embodiment can automatically adjust an optical element. In the optical device Db according to this embodiment, by controlling the wavefront state θ of the wavefront control device 3 once, even a user having no sufficient knowledge of adjustment of an optical element can continuously acquire an operation image OP having imaging performance desired by the user.

In addition, the optical device Db according to this embodiment has the learning unit 74E. The learning unit 74E has learned the relationship between an image signal IS and a phase of the wavefront in the pupil of the imaging optical system OS in advance. In addition, a control unit (the control device 7E in this example) controls the wavefront control device 3 on the basis of a learning result acquired by the learning unit 74E and an image signal IS output by the imaging element 6.

By employing such a configuration, the optical device Db according to this embodiment can control the wavefront control device 3 on the basis of a learning result acquired by learning the relationship between an image signal IS and a phase of the wavefront in the pupil of the imaging optical system OS in advance and the image signal IS output by the imaging element 6, and thus the imaging performance of the optical device can be improved more than that of a case not based on a learning result.

In addition, in the learning process according to each of the embodiments described above, deep learning may be used. In a case in which deep learning is used in the learning process, the intensity of light collected by the imaging element 6 is used as a value input to an input layer of a neural network of the deep learning, each wavefront state $\theta$ is used as a weighting factor, and a value output from an output layer corresponds to an imaging performance evaluation value Y.

In a case in which deep learning is used in the learning process, a learning result acquired by learning the relationship between an image signal IS and a phase of the wavefront in the pupil of the imaging optical system OS in advance is a result that is acquired through the deep learning.

In addition, in each of the embodiments described above, although a case in which the control device 7B and the control device 7E use a learning result for controlling the wavefront state $\theta$ of the wavefront control device 3 has been described, the configuration is not limited thereto. The control device 7B and the control device 7E may control the wavefront state $\theta$ of the wavefront control device 3 on the basis of an image signal IS output from the imaging element 6.

For example, the control device 7B and the control device 7E may sequentially acquire image signals IS from the imaging element 6 and search for a wavefront state $\theta$ in which the imaging performance PY of a specific area RC of an image P becomes higher than that at the current time.

In addition, in each of the embodiments described above, although a case in which a value to be calculated by the evaluation value calculating unit 741 as the imaging performance PY is determined in advance has been described, the configuration is not limited thereto. A value to be calculated by the evaluation value calculating unit 741 as the imaging performance PY may be set by a user of the imaging system S (the optical device D).

In addition, in each of the embodiments described above, although a case in which the optical system condition A is acquired from the imaging optical system OS or the optical simulation device 7C as the optical system condition information LS or the learning optical system condition information LLS by the learning device 7A, the control device 7B, the learning device 7Aa, and the control device 7E has been described, the configuration is not limited thereto. The optical system condition A may be set by the learning device 7A, the control device 7B, the learning device 7Aa, and the control device 7E and be supplied to the imaging optical system OS or the optical simulation device 7C.

In addition, parts of the optical device D and the optical device Db according to the embodiment described above, for example, the learning devices 7A and 7Aa and the control devices 7B and 7E may be realized by a computer. In such a case, a program for realizing this control function may be recorded on a computer-readable recording medium, and the control function may be realized by causing a computer system to read and execute the program recorded on this recording medium. The "computer system" described here is a computer system built in the optical device D and the optical device Db and includes an OS and hardware such as peripherals and the like. In addition, the "computer-readable recording medium" is a portable medium such as a flexible disc, a magneto-optical disk, a ROM, a CD-ROM, or the like or a storage device such as a hard disk built into a computer system or the like. Furthermore, the "computer-readable recording medium" may include a medium that dynamically stores a program for a short time such as a communication line in a case in which a program is transmitted through a network such as the Internet or a communication line such as a telephone line and a medium that stores a program for a predetermined time such as an internal volatile memory of the computer system serving as a server or a client in that case. The program described above may be a program for realizing some of the functions described above or may be a program that can realize the function described above by being combined with a program that has already been recorded in the computer system in advance.

Some or all of the learning devices 7A and 7Aa and the control devices 7B and 7E according to the embodiments described above may be realized as an integrated circuit such as a large scale integration (LSI). Each of the functional blocks of the learning devices 7A and 7Aa and the control devices 7B and 7E may be individually configured as a processor, or some of all thereof may be integrated and configured as a processor. The technique for integrating circuits is not limited to the LSI, and each functional block may be realized by a dedicated circuit or a general-purpose processor. In addition, in a case in which a technology for integrating circuits replacing the LSI appears in accordance with advancement of semiconductor technologies, an integrated circuit utilizing such technology may be used.

As above, although the embodiments of the present invention have been described in detail with reference to the drawings, specific configurations are not limited to those described above, and various changes in design and the like can be made within a range not departing from the concept of the present invention.

[Reference Signs List]

| S, Sb | Imaging system |
| D, Db | Optical device |
| D, OS | Imaging optical system |
| 1 | Subject |
| 2 | Objective lens system |
| 3 | Wavefront control device |
| 4 | Diaphragm |
| 5 | Imaging lens system |
| 6 | Imaging element |
| 7 | Computer |
| 8 | Display unit |
| 9 | Input unit |
| 7A, 7Aa | Learning device |
| 71A | Learning image signal acquiring unit |
| 72Aa | Learning wavefront control signal generating unit |
| 74, 74E | Learning unit |
| 740 | Feature quantity calculating unit |
| 741 | Evaluation value calculating unit |
| 742 | Relation learning unit |
| 743, 743E | Wavefront state estimating unit |
| 744, 744E | Wavefront state table generating unit |
| 745E | Solution estimation range managing unit |
| 75A, 75Aa | Optical system condition acquiring unit |
| 7C | Optical simulation device |
| 70C | Imaging optical system simulating unit |
| 71C | Wavefront control simulating unit |
| 7D | Retina image database |
| 7B, 7E | Control device |
| 70B | Area setting information acquiring unit |
| 71E | Image signal acquiring unit |
| 72B, 72E | Wavefront control signal generating unit |
| 73B, 73E | Storage unit |
| 75B, 75E | Optical system condition information acquiring unit |
| 76E | Visibility signal acquiring unit |
| 77E | Mode setting managing unit |

**Claims**

1. An optical device comprising:

an imaging optical system configured to form an image of a subject;
an imaging element configured to output an image signal of the image of the subject that is formed by the imaging optical system;
a spatial modulation element configured to change a phase of a wavefront in a pupil of the imaging optical system; and
a control unit configured to control the spatial modulation element on the basis of the image signal output by the imaging element.

2. The optical device according to claim 1, wherein the spatial modulation element is disposed at a position conjugate with the pupil of the imaging optical system.

3. The optical device according to claim 1 or 2, wherein the control unit is configured to perform control of the spatial modulation element such that the phase of the wavefront of an area in the pupil corresponding to a specific area in the image acquired from the image signal is changed.

4. The optical device according to claim 3, further comprising an input unit configured to accept an input from a user, wherein the specific area is set on the basis of the input accepted by the input unit.

5. The optical device according to any one of claims 1 to 4, wherein the control unit is configured to fix a state in which the spatial modulation element is controlled on the basis of a predetermined condition.

6. The optical device according to any one of claims 1 to 5, further comprising a learning unit that has learned a relationship between the image signal and the phase of the wavefront in the pupil in advance, wherein the control unit is configured to control the spatial modulation element on the basis of a learning result acquired by the learning unit and the image signal output by the imaging element.

7. The optical device according to claim 6, wherein the learning result is a result acquired through deep learning.

8. A program for causing a computer of an optical device, which includes an imaging optical system configured to form an image of a subject, an imaging element configured to output an image signal of the image of the subject that is formed by the imaging optical system, and a spatial modulation element configured to change a phase of a wavefront in a pupil of the imaging optical system, to execute:

an acquisition step of acquiring the image signal output by the imaging element; and
a control step of controlling the spatial modulation element on the basis of the image signal acquired in the acquisition step.

9. A control device comprising:
a control unit configured to control a spatial modulation element changing a phase of a wavefront in a pupil of an imaging optical system, which forms an image of a subject, on the basis of an image signal that is a signal acquired by imaging the image of the subject formed by the imaging optical system using an imaging element.

10. The control device according to claim 9, wherein the spatial modulation element is disposed at a position conjugate with the pupil of the imaging optical system.

11. The control device according to claim 9 or 10, wherein the control unit is configured to perform control of the spatial modulation element such that a phase of the wavefront of an area in the pupil corresponding to a specific area in the image acquired from the image signal is changed.

12. The control device according to claim 11, wherein the specific area is an area set by a user.

13. The control device according to any one of claims 9 to 12, wherein the control unit is configured to fix a state in which the spatial modulation element is controlled on the basis of a predetermined condition.

14. The control device according to any one of claims 9 to 13, wherein the spatial modulation element is controlled on the basis of a learning result acquired by a learning unit that has learned a relationship between the image signal and the phase of the wavefront in the pupil in advance and the image signal output by the imaging element.

15. The control device according to claim 14, wherein the learning result is a result acquired through deep learning.

16. An imaging system comprising:

the control device according to any one of claims 9 to 15;
the imaging optical system;
the imaging element configured to output the image signal of the image of the subject formed by the imaging optical system; and
the spatial modulation element.

17. A program causing a computer to execute:

an acquisition step of acquiring an image signal that is a signal acquired by imaging an image of a subject formed by an imaging optical system forming the image of the subject using an imaging element; and
a control step of controlling a spatial modulation element changing a phase of a wavefront in a pupil of the imaging optical system on the basis of the image signal acquired in the acquisition step.

EP 3 895 599 A1

# FIG. 1

FIG. 2

<u>P</u>

R

# FIG. 3

# FIG. 4

LEARNING DEVICE — 7A

- LEARNING IMAGE SIGNAL ACQUIRING UNIT — 71A
- LEARNING WAVEFRONT CONTROL SIGNAL GENERATING UNIT — 72A
- OPTICAL SYSTEM CONDITION INFORMATION ACQUIRING UNIT — 75A

LEARNING UNIT — 74
- FEATURE QUANTITY CALCULATING UNIT — 740
- EVALUATION VALUE CALCULATING UNIT — 741
- RELATION LEARNING UNIT — 742
- WAVEFRONT STATE ESTIMATING UNIT — 743
- WAVEFRONT STATE TABLE GENERATING UNIT — 744

LIS → IMAGING ELEMENT — 6

OPTICAL SIMULATION DEVICE — 7C
- IMAGING OPTICAL SYSTEM SIMULATING UNIT — 70C
- WAVEFRONT CONTROL SIMULATING UNIT — 71C

LLS

LWS

RI ← RETINA IMAGE DATABASE — 7D

T → CONTROL DEVICE — 7B

EP 3 895 599 A1

FIG. 5

7B

CONTROL DEVICE

RS

70B

AREA SETTING INSTRUCTION
ACQUIRING UNIT

9

INPUT UNIT

75B

OPTICAL SYSTEM CONDITION
INFORMATION ACQUIRING UNIT

72B

WAVEFRONT CONTROL SIGNAL
GENERATING UNIT

LS

OS

IMAGING
OPTICAL SYSTEM

73B

STORAGE UNIT

T

WAVEFRONT STATE TABLE

WS

3

WAVEFRONT
CONTROL DEVICE

## FIG. 6

```
        ( START )
            │
            ▼
┌──────────────────────────┐
│ GENERATE WAVEFRONT STATE │──S100
└──────────────────────────┘
            │
            ▼
┌──────────────────────────┐
│      SELECT OPTICAL      │──S110
│     SYSTEM CONDITION     │
└──────────────────────────┘
            │
            ▼
┌──────────────────────────┐
│   SET OPTICAL SYSTEM DATA │──S120
└──────────────────────────┘
            │
            ▼
┌──────────────────────────┐
│ EXECUTE OPTICAL SIMULATION│──S130
└──────────────────────────┘
            │
            ▼
┌──────────────────────────┐
│ CALCULATE IMAGING PERFOR-│──S140
│  MANCE EVALUATION VALUE  │
└──────────────────────────┘
            │
            ▼
┌──────────────────────────┐
│   LEARN RELATION BETWEEN │
│ WAVEFRONT STATE AND IMAGING│──S150
│  PERFORMANCE EVALUATION  │
│   VALUE FOR SPECIFIC AREA │
└──────────────────────────┘
            │
            ▼
┌──────────────────────────┐
│  ESTIMATE WAVEFRONT STATE │
│  IN WHICH IMAGING PERFOR-│──S160
│   MANCE EVALUATION VALUE │
│ IS MAXIMUM IN SPECIFIC AREA│
└──────────────────────────┘
            │
            ▼
┌──────────────────────────┐
│     GENERATE WAVEFRONT   │──S170
│       STATE TABLE        │
└──────────────────────────┘
            │
            ▼
         ( END )
```

## FIG. 7

| ZOOM MAGNIFICATION | 2 x |
|---|---|
| PRELIMINARY LENS | TYPE A |
| SUBJECT | NAVARRO EYE MODEL |

# FIG. 8

| PIXEL OF WAVEFRONT CONTROL DEVICE | 1 | 2 | ... | 36 |
|---|---|---|---|---|
| PHASE DELAY [λ] | 0.5 | 0.25 | ... | 1 |

# FIG. 9

| AREA | R1 | R2 | ... | R25 |
|---|---|---|---|---|
| psf(strehlratio) | 0.5 | 0.3 | ... | 0.6 |

| AREA | R1 | R2 | ... | R25 |
|---|---|---|---|---|
| WEIGHTING FACTOR | 1 | 5 | ... | 2 |

# FIG. 10

START

ACQUIRE AREA SETTING INSTRUCTION — S200

ACQUIRE OPTICAL SYSTEM CONDITION INFORMATION — S210

SELECT WAVEFRONT STATE DATA — S220

CONTROL WAVEFRONT STATE — S230

END

FIG. 11

LEARNING DEVICE 7Aa

LEARNING IMAGE SIGNAL ACQUIRING UNIT 71A

LEARNING WAVEFRONT CONTROL SIGNAL GENERATING UNIT 72Aa

OPTICAL SYSTEM CONDITION INFORMATION ACQUIRING UNIT 75Aa

LEARNING UNIT 74

FEATURE QUANTITY CALCULATING UNIT 740

EVALUATION VALUE CALCULATING UNIT 741

RELATION LEARNING UNIT 742

WAVEFRONT STATE ESTIMATING UNIT 743

WAVEFRONT STATE TABLE GENERATING UNIT 744

LIS — IMAGING ELEMENT 6

LLS — IMAGING OPTICAL SYSTEM OS

LWS — WAVEFRONT CONTROL DEVICE 3

RI — RETINA IMAGE DATABASE 7D

T — CONTROL DEVICE 7B

EP 3 895 599 A1

# FIG. 12

```
           ┌─────────┐
           │  START  │
           └─────────┘
                │
    ┌───────────────────────┐
    │ GENERATE WAVEFRONT STATE │─── S300
    └───────────────────────┘
                │
    ┌───────────────────────┐
    │    SELECT OPTICAL      │─── S310
    │   SYSTEM CONDITION     │
    └───────────────────────┘
                │
    ┌───────────────────────┐
    │ IMAGE POINTS AND PATTERN │─── S320
    └───────────────────────┘
                │
    ┌───────────────────────┐
    │  CALCULATE IMAGING PER- │─── S330
    │ FORMANCE EVALUATION VALUE │
    └───────────────────────┘
                │
    ┌───────────────────────┐
    │ LEARN RELATION BETWEEN │
    │ WAVEFRONT STATE AND IMAGING │─── S340
    │ PERFORMANCE EVALUATION │
    │  VALUE IN SPECIFIC AREA │
    └───────────────────────┘
                │
    ┌───────────────────────┐
    │ ESTIMATE WAVEFRONT STATE │
    │ IN WHICH IMAGING PERFOR- │─── S350
    │ MANCE EVALUATION VALUE IS │
    │ MAXIMUM FOR SPECIFIC AREA │
    └───────────────────────┘
                │
    ┌───────────────────────┐
    │  GENERATE WAVEFRONT    │─── S360
    │     STATE TABLE        │
    └───────────────────────┘
                │
           ┌─────────┐
           │   END   │
           └─────────┘
```

# FIG. 13

**CONTROL DEVICE** (7E)

**AREA SETTING INSTRUCTION ACQUIRING UNIT** (70B)

**IMAGE SIGNAL ACQUIRING UNIT** (71E)

**WAVEFRONT CONTROL SIGNAL GENERATING UNIT** (72E)

**OPTICAL SYSTEM CONDITION INFORMATION ACQUIRING UNIT** (75B)

**VISIBILITY SIGNAL ACQUIRING UNIT** (76E)

**MODE SETTING MANAGING UNIT** (77E)

**LEARNING UNIT** (74E)

**FEATURE QUANTITY CALCULATING UNIT** (740)

**EVALUATION VALUE CALCULATING UNIT** (741)

**RELATION LEARNING UNIT** (742)

**WAVEFRONT STATE ESTIMATING UNIT** (743E)

**WAVEFRONT STATE TABLE GENERATING UNIT** (744E)

**SOLUTION ESTIMATION RANGE MANAGING UNIT** (745E)

**STORAGE UNIT** (73E)

**PRESET WAVEFRONT STATE TABLE** (TP)

**MODE ESTIMATION INFORMATION** (ST)

**ADAPTIVE WAVEFRONT STATE TABLE** (TA)

RS / MS / VS → **INPUT UNIT** (9)

IS → **IMAGING ELEMENT** (6)

LS → **IMAGING OPTICAL SYSTEM** (OS)

WS → **WAVEFRONT CONTROL DEVICE** (3)

EP 3 895 599 A1

# FIG. 14

```
                    ┌──────────┐
                    │  START   │
                    └──────────┘
                         │
                         ▼
            ┌─────────────────────────┐
            │   ACQUIRE  AREA         │   S400
            │ SETTING  INSTRUCTION    │
            └─────────────────────────┘
                         │
                         ▼
            ┌─────────────────────────┐
            │ ACQUIRE OPTICAL SYSTEM  │   S410
            │  CONDITION  INFORMATION │
            └─────────────────────────┘
                         │
                         ▼
            ┌─────────────────────────┐
            │  SET  WAVEFRONT  STATE  │   S415
            │   SEARCH  RANGE         │
            └─────────────────────────┘
                         │
                         ▼
                      ╱────────╲       S420
                    ╱    HAS     ╲         NO
                   ╱  "PRESET"  BEEN SET   ╲──────────────────────┐
                    ╲      ?     ╱                                 │
                      ╲────────╱                                  ▼
                         │ YES     S430              ┌──────────────────────────┐ S480
                         ▼                           │  PERFORM ADAPTIVE        │
            ┌─────────────────────────┐              │  LEARNING  PROCESS       │
            │ SELECT PRESET WAVEFRONT │              └──────────────────────────┘
            │   STATE  TABLE          │                         │
            └─────────────────────────┘                         ▼           S490
                         │                           ┌──────────────────────────┐
                         │◄──────────────────────────│  SELECT  ADAPTIVE        │
                         │                           │ WAVEFRONT  STATE  TABLE  │
                         ▼                           └──────────────────────────┘
            ┌─────────────────────────┐
            │ CONTROL WAVEFRONT STATE │   S440
            └─────────────────────────┘
                         │
                         ▼
            ┌─────────────────────────┐
            │ ACQUIRE VISIBILITY SIGNAL│  S450
            └─────────────────────────┘
                         │
                         ▼
                      ╱────────╲        S460
                    ╱    HAS     ╲          NO
                   ╱ "VISIBLE"  DETERMINED  ╲───────────────┐
                    ╲  FOR USER ?  ╱                        ▼           S500
                      ╲────────╱                 ┌──────────────────────┐
                         │ YES     S470          │  SELECT  "PRESET"    │
                         ▼                       │  OR  "ADAPTIVE"      │
            ┌─────────────────────────┐          └──────────────────────┘
            │ MAINTAIN WAVEFRONT STATE│
            └─────────────────────────┘
                         │
                         ▼
                    ┌──────────┐
                    │   END    │
                    └──────────┘
```

# FIG. 15

```
         ┌─────────┐
         │  START  │
         └─────────┘
              │
              ▼
  ┌───────────────────────┐
  │ UPDATE  WAVEFRONT  STATE │ ⌒ S600
  │     SEARCH  RANGE      │
  └───────────────────────┘
              │
              ▼
  ┌───────────────────────┐
  │ ESTIMATE  WAVEFRONT  STATE │
  │ FOR  WHICH  IMAGING  PERFOR- │ ⌒ S610
  │ MANCE  EVALUATION  VALUE  IS │
  │ MAXIMUM  IN  SPECIFIC  AREA │
  └───────────────────────┘
              │
              ▼
  ┌───────────────────────┐
  │    GENERATE  ADAPTIVE    │ ⌒ S620
  │  WAVEFRONT  STATE  TABLE │
  └───────────────────────┘
              │
              ▼
         ┌─────────┐
         │   END   │
         └─────────┘
```

## INTERNATIONAL SEARCH REPORT

| International application No. |
| --- |
| PCT/JP2019/039521 |

A. CLASSIFICATION OF SUBJECT MATTER
Int.Cl. A61B3/12(2006.01)i, A61B3/13(2006.01)i

According to International Patent Classification (IPC) or to both national classification and IPC

B. FIELDS SEARCHED

Minimum documentation searched (classification system followed by classification symbols)
Int.Cl. A61B3/00-3/18

Documentation searched other than minimum documentation to the extent that such documents are included in the fields searched
Published examined utility model applications of Japan       1922-1996
Published unexamined utility model applications of Japan     1971-2019
Registered utility model specifications of Japan             1996-2019
Published registered utility model applications of Japan     1994-2019

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
| --- | --- | --- |
| X<br><br>A | JP 2018-528830 A (SIMON FRASER UNIVERSITY) 04 October 2018, paragraphs [0036]-[0042], fig. 3, 5(A)<br>& WO 2017/044969 A1, pages 12, 13, fig. 3, 5(A) & US 2018/0055355 A1 & CN 108135466 A & KR 10-2018-0059820 A | 1, 3-4, 8-9, 11-12, 16-17<br><br>2, 10 |
| A | JP 2016-067764 A (NIDEK CO., LTD.) 09 May 2016, paragraphs [0098]-[0100]<br>(Family: none) | 1-4, 8-12, 16-17 |

☐ Further documents are listed in the continuation of Box C.    ☐ See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| 11.12.2019 | 24.12.2019 |

| Name and mailing address of the ISA/<br>Japan Patent Office<br>3-4-3, Kasumigaseki, Chiyoda-ku,<br>Tokyo 100-8915, Japan | Authorized officer<br><br><br>Telephone No. |
| --- | --- |

Form PCT/ISA/210 (second sheet) (January 2015)

# INTERNATIONAL SEARCH REPORT

| International application No. |
| --- |
| PCT/JP2019/039521 |

**Box No. II      Observations where certain claims were found unsearchable (Continuation of item 2 of first sheet)**

This international search report has not been established in respect of certain claims under Article 17 (2)(a) for the following reasons:

1. ☐ Claims Nos.:
because they relate to subject matter not required to be searched by this Authority, namely:

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**Box No. III      Observations where unity of invention is lacking (Continuation of item 3 of first sheet)**

This International Searching Authority found multiple inventions in this international application, as follows:
[see extra sheet]

1. ☐ As all required additional search fees were timely paid by the applicant, this international search report covers all searchable claims.

2. ☐ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☒ No required additional search fees were timely paid by the applicant.   Consequently, this international search report is restricted to the invention first mentioned in the claims; it is covered by claims Nos.:
Claims 1-4, 8-12, 16-17

**Remark on Protest**      ☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet (2)) (January 2015)

| INTERNATIONAL SEARCH REPORT | International application No. |
|---|---|
| | PCT/JP2019/039521 |

(Continuation of Box No. III)
(Invention 1) Claims 1-4, 8-12, and 16-17
    Document 1 discloses a sensorless adaptive optics imaging system capable of acquiring an OCT image, the system being
    (1) provided with an optical delivery unit (equivalent to the "spatial modulation element") having an adaptive optics element MAL, which is a wavefront modifier, and
    (2) configured to control the optical delivery unit such that the wavefront coefficient is optimized by analyzing an image (equivalent to the "image signal") provided in a selected location.
    In addition, it is found in fig. 3 of document 1 that, when an OCT image is acquired by this system, an imaging optical system is utilized.
    In view of this, claim 1, which lacks novelty in light of document 1, does not have a special technical feature. However, claim 2 that depends from claim 1 has the special technical feature wherein "the spatial modulation element is disposed in a position conjugated to the pupil of the imaging optical system".
    It can be said that the invention of claims 8, 9, 16, and 17 is substantially identical to or similarly closely related to the invention of claim 1.
    Accordingly the invention of claims 1, 2, 8, 9, 16, and 17 and the invention of claims 3, 4, and 10-12 that depend from any of claims 1, 8, and 9 are classified as invention 1 that serves as a main invention.

(Invention 2) Claims 5 and 13
    Claim 5 cannot be said to have the same or corresponding technical features between this claim and claim 2 classified as invention 1.
    In addition, although claim 5 depends from claim 1 classified as invention 1, the technical link is weak between the technical feature of claim 1 and the added technical feature relative to claim 1 wherein "the control unit fixes the state where the spatial modulation element has been controlled on the basis of a predetermined condition". Thus, it is not considered that claim 5 has a link of invention to claim 1.
    Furthermore, claim 5 is not substantially identical to or similarly closely related to any of the claims classified as invention 1.
    Accordingly the invention of claim 5 and the invention of claim 13 that is an invention substantially identical to or similarly closely related to claim 5 cannot be identified as invention 1, and thus are classified as invention 2.

(Invention 3) Claims 6-7 and 14-15
    Claim 6 cannot be said to have the same or corresponding technical features between this claim and claim 2 classified as invention 1 or claim 5 classified as invention 2.
    In addition, although claim 6 depends from claim 1 classified as invention 1, the technical link is weak between the technical feature of claim 1 and the added technical feature relative to claim 1 of "having a learning unit in which the connection between the image signal and the phase of the wavefront in the pupil is beforehand learned,
    wherein the control unit controls the spatial modulation element on the basis of the learning result of the learning unit and the image signal output by the imaging element". Thus, it is not considered that claim 6 has a link of invention to claim 1.
    Furthermore, claim 6 is not substantially identical to or similarly closely related to any of the claims classified as invention 1 or 2.
    Accordingly the invention of claim 6, the invention of claim 14 that is an invention substantially identical to or similarly closely related to claim 6, and in addition, the invention of claims 7 and 15 that depend from any of these claims cannot be identified as either of inventions 1 and 2, and thus are classified as invention 3.

Form PCT/ISA/210 (extra sheet) (January 2015)

**EP 3 895 599 A1**

**Patent documents cited in the description**

- JP 2018230771 A
- JP 2015072303 A